Europäisches Patentamt

European Patent Office

Office européen des brevets

(11) Numéro de publication: **0 550 779 B1**

# FASCICULE DE BREVET EUROPEEN

(45) Date de publication du fascicule du brevet: **18.10.95**    (51) Int. Cl.6: **A61K 7/48**, A61K 31/23

(21) Numéro de dépôt: **92100208.5**

(22) Date de dépôt: **08.01.92**

(54) **Composition cosmétique ou dermatologique contenant un diol diester.**

(43) Date de publication de la demande:
**14.07.93 Bulletin 93/28**

(45) Mention de la délivrance du brevet:
**18.10.95 Bulletin 95/42**

(84) Etats contractants désignés:
**AT BE CH DE DK ES FR GB IT LI NL SE**

(56) Documents cités:
**FR-A- 2 281 916**

**WORLD PATENTS INDEX LATEST Section Ch,
Week 8310, Derwent Publications Ltd., London, GB; Class B, AN 83-23820K**

(73) Titulaire: **SOCIETE DES PRODUITS NESTLE
S.A.
Case postale 353
CH-1800 Vevey (CH)**

(72) Inventeur: **Traitler, Helmut
5, Ch.Entre-deux-Villes
CH-1802 Corseaux (CH)**
Inventeur: **Viret, Jean-Louis
La Bruyère A. Fontanivent
CH-1817 Brent (CH)**

**EP 0 550 779 B1**

## Description

La présente invention concerne une composition cosmétique ou dermatologique contenant un diester de 2,3-butanediol et d'acide gras en C16-C22 à titre d'agent de viscosité et d'émollient.

FR-A-2 281 916 décrit une composition cosmétique contenant un diol monoester comme agent onctueux.

Dans les compositions cosmétiques ou dermatologiques contenant une phase grasse du type des émulsions ou anhydres, on utlise fréquemment des esters, hydrocarbures et glycérides à titre d'agents de viscosité et d'émollients.

Les qualités principales requises pour ces composés sont multiples:
- ils doivent être miscibles, pratiquement en toute proportion avec les esters, corps gras et vitamines liposolubles d'emploi courant en cosmétique et en galénique,
- ils doivent être de préférence incolores et pratiquement inodores,
- leur pouvoir dissolvant doit leur permettre de servir de véhicule aux principes actifs liposolubles,
- ils doivent être de préférence facilement émulsionnables,
- ils ne doivent pas influencer négativement les propriétés rhéologiques, notamment la viscosité et la thixotropie des produits finis et
- ils doivent avoir de bonnes caractéristiques sensorielles ou organoleptiques, c'est à dire conférer aux produits finis une sensation agréable sur la peau, par exemple par leur faculté d'étalement.

Nous avons trouvé que les diesters de 2,3-butane diol et d'acides gras en C16-C22 possédaient les qualités énoncées précédemment à un degré remarquable.

Les composés ci-dessus entrant dans la composition selon l'invention et leur procédé de préparation sont décrits dans la demande de brevet EP-A-0465689.

La nature du ou des acides gras constitutifs des esters et leurs proportions sont dictées par les propriétés physico-chimiques, de stabilité chimique et sensorielles souhaitées dans le cadre d'une application cosmétique particulière. Dans cet ordre d'idées, les acides gras sont de préférence les saturés palmitique, myristique, stéarique, laurique et les mono- ou di-insaturés, oléique et linoléique, avec une prédominance des oléique, stéarique et palmitique.

Ainsi, si l'ester comporte par exemple en prédominance l'acide oléique, il se présentera sous la forme d'une huile à la température ambiante, par exemple 2O°C avec des propriétés essentiellement émollientes, c est à dire adoucissantes et amollissantes de la peau. Si l'ester est à prédominance d'acides gras saturés, par exemple stéarique et palmitique, il se présentera sous forme de solide cristallisé à 20°C et constituera en sus un agent de viscosité du fait de sa consistance.

Les composés préférés précédents peuvent être utilisés dans pratiquement toutes les préparations cosmétiques ou galéniques aqueuses telles que par exemple les crèmes, laits, shampooings sous forme d'émulsions eau-dans-l'huile ou huile-dans-l'eau ou anhydres telles que par exemple les huiles de bain, de massage, solaires, les baumes, fonds de teint et rouges à lèvres.

Une composition selon l'invention contient de préférence 0,5 à 80% en poids de diol diester tel que défini précédemment.

Dans une telle composition, la phase grasse peut contenir des huiles animales, végétales, minérales ou synthétiques. Elle peut contenir également des cires, des alcools à longue chaîne, des agents épaississants ou gélifiants. Lorsqu'il s'agit d'une émulsion, une composition cosmétique peut contenir 1 à 20% en poids d'un agent émulsifiant.

Dans une composition cosmétique anhydre, la phase grasse peut contenir 10 à 80% en poids, et de préférence 10 à 40% en poids de diol diester par rapport au poids total de la composition. En outre, elle peut contenir des huiles et une proportion relativement élevée, par exemple de 5 à 30% en poids de cires. Elle peut se présenter par exemple sous forme d'huile antisolaire (auquel cas elle contient un filtre solaire absorbant les rayons ultra-violets).

Une composition selon l'invention peut contenir en outre divers additifs, notamment des agents colorants, des parfums, des conservateurs, des filtres UV, des agents nacrants et des charges minérales ou organiques. Elle contient avantageusement des antioxydants, à raison de 0,02 à 0,2% en poids.

Les exemples ci-après illustrent l'invention. Dans ceux-ci, les parties et pourcentages sont pondéraux sauf indication contraire.

Dans ces exemples, la nomencalutre utilisée est celle de la "Cosmetic, Toiletery and Flagrance Association, Inc. Washington DC" (CTFA), mise à part la traduction en français.

Pour fabriquer les émulsions, on mélange séparément les composants des phases lipidiques A et le cas échéant B et on les chauffe à 70°C, puis on incorpore le cas échéant la phase B dans la phase A. On prépare la phase aqueuse C en mélangeant ses composants et on la chauffe à 70°C. On ajoute la ou les

2

phase(s) lipidique(s) A et le cas échéant B à la phase aqueuse C (dans le cas des émulsions huile-dans-l'eau) à 70°C en brassant à vitesse moyenne [dans le cas des émulsions eau-dans-l'huile on ajoute la phase aqueuse à la ou aux phase(s) lipidique(s)]. On homogénéise le mélange des deux phases puis on le brasse à environ 100 t/min et on le laisse refroidir à 45-50°C pour les émulsions eau-dans-l'huile et à 35-40°C pour les émulsions huile-dans-l'eau.

A cette température, on ajoute le cas échéant les additifs, puis on continue à refroidir jusqu'à la température ambiante en brassant lentement et on arrête le brassage lorsque le produit est semi-fluide (à environ 25°C).

Les produits anhydres sont obtenus de la même manière mais sans homogénéisation, par mélange à chaud (environ 70°C), puis refroidissement progressif sous brassage lent.

Ce mode opératoire est appliqué dans les exemples par défaut. Dans le cas où un mode opératoire différent est suivi, celui-ci est indiqué spécifiquement.

Exemples 1-2

Dans ces exemples, on utilise une méthode sensorielle pour évaluer les caractéristiques organoleptiques des diol diesters lors de leur application sur la peau à partir d'une lotion sous forme d'une émulsion huile-dans-l'eau.

Par ailleurs, on évalue la viscosité dynamique (au cisaillement maximum, en m Pa. s) et la thixotropie des lotions. La thixotropie est déterminée à partir de la courbe d'écoulement (rhéogramme, force de cisaillement fonction du gradient de vitesse de cisaillement) comme la surface entre la courbe ascendante et la courbe descendante, exprimée en Pa. s. On effectue les mesures de viscosité et de thixotropie à 20°C avec un appareil Rheomat 115® de Contraves.

Méthode

A. Définition

Cette méthode permet de quantifier numériquement par un indice (IDT, indice du toucher) les différentes caractéristiques qui définissent le toucher des produits cosmétiques et galéniques.

L'IDT comprend trois paramètres:

1. Le toucher initial composé :
   - d'une note de texture de 1 (trop aqueuse, pauvre) à 5 (onctueuse, très riche) et
   - d'une note de glissement de 1 (freine trop) à 5 (glisse bien),
   la cotation constituant la moyenne des deux notes.

2. Le toucher intermédiaire pendant l'étalement comprenant :
   - une note d'étalement de 1 (freine trop) à 5 (glisse bien),
   - une note déterminant la cassure ou le changement de texture de 1 (devenant franchement aqueuse) à 5 (aucun changement),
   - une note de caractère collant de 1 (trop collant) à 5 (pas collant),
   - une note de vitesse de pénétration de 1 (peu satisfaisante) à 5 (optimale) et
   - une note de degré de pénétration de 1 (inexistante ou mauvaise) à 5 (très bonne),
   la cotation constituant la moyenne des cinq notes.

3. Le toucher final après évaporation complète des composés volatils comprenant :
   - une note d'impression au frottement de 1 (freine trop) à 5 (glisse bien),
   - une note caractérisant le film lipidique résiduel sur la peau de 1 (inexistant, peau sèche) à 5 (riche, peau bien nourrie) et
   - une note de douceur de la peau de 1 (rugueuse) à 5 (veloutée, très douce),
   la cotation étant la somme des trois notes.

L'IDT est exprimé par la cotation du toucher initial / la somme de la cotation du toucher intermédiaire et de la cotation du toucher final.

B. Partie expérimentale

Le test est basé sur la comparaison entre une formule expérimentale contenant la substance à tester et une formule témoin servant de référence pour attribuer les cotations, la formule témoin ayant la composition suivante :

|  |  | % |
|---|---|---|
| **PHASE A (lipidique)** |  | **10,1** |
| PEG-10 isocéthyl ether |  |  |
| monostéarate | 4,5 |  |
| Stéareth-21 | 1,5 |  |
| Stéarate de glycérol | 2,6 |  |
| Alcool cétéarylique | 1,5 |  |
| **PHASE B (lipidique)** |  | **6,3** |
| Huile de paraffine | 6,0 |  |
| Carbomer 934 (polymère |  |  |
| d'acide acrylique polyréticulé) | 0,3 |  |
| **PHASE C (aqueuse)** |  | **81,8** |
| Eau | 76,7 |  |
| Glycérol | 5,0 |  |
| Ethylènediaminetétra- |  |  |
| acétate (EDTA) | 0,1 |  |
| **ADDITIFS** |  | **1,8** |
| Phénoxyparaben | 0,6 |  |
| Silméthicone | 0,1 |  |
| Triméthamine (solution |  |  |
| aqueuse à 30%) | 0,8 |  |
| Parfum | 0,3 |  |
|  |  | **100** |

Cette formule (ci-après "comparaison 1") a été élaborée de façon à obtenir une valeur IDT de référence; cette valeur moyenne pourra augmenter ou diminuer en rapport avec la qualité de la ou des substances à tester qui est (sont) incorporée(s) à cette formule en lieu et place des 6% d'huile de paraffine. Dans le cas de l'exemple 2, on incorpore 10% du composé et on compense en supprimant le stéarate de glycérol et l'alcool cétéarylique.

Les échantillons de lotions à tester sont soumis aux expérimentateurs par groupe de 4 à 6, ce qui correspond au nombre limite qui peut être testé par séance d'essai.

Le produit est appliqué sur les intérieurs des avants-bras. La quantité de produit à appliquer doit être la même pour chaque essai, c'est-à-dire environ 0,2 g.

4

Les échantillons sont testés à "l'aveugle". Chaque expérimentateur prend note de ses sensations, depuis l'application du produit jusqu'à son séchage sur la peau. La notation se fait dans l'ordre du questionnaire. Une fois le test réalisé, le nom du produit est révélé aux expérimentateurs.

Cette méthode permet de quantifier le toucher de n'importe quel produit cosmétique qui sera appliqé sur la peau et de le faire de façon raisonnablement reproductible, et ceci aussi bien dans le cas où l'expérimentateur répète ses propres essais à divers intervalles de temps que lorsqu'il répète les essais d'un autre expérimentateur.

Les produits à tester sont :

Exemple 1 :

le produit de l'exemple 1 de la demande de brevet EP-A-0465689 dont les propriétés physico-chimiques sont :

| - Aspect | liquide huileux incolore et pratiquement inodore |
| --- | --- |
| - Indice de réfraction | 1,4635 |
| - Acides gras libres (%) | 0,05 - 1 |
| - Viscosité dynamique (mPa.s) | 49 |
| - Densité à 22 °C | 0,895 |

Exemple 2 :

le produit de l'exemple 3 de la demande de brevet EP-A-0465689 dont les propriétés physico-chimiques sont :

| - Aspect à 20 °C | solide cristallisé de couleur blanche |
| --- | --- |
| - Aspect à 50 °C | liquide huileux incolore et pratiquement inodore |
| - Point de fusion | autour de 40 °C |

A titre de comparaison, on met en oeuvre en tant qu'émollients d'autres produits que l'huile de paraffine, qui représentent les différentes classes d'émollients couramment utilisés en cosmétique pour cet usage :

| - Comparaison 2 | octyl palmitate, |
| --- | --- |
| - Comparaison 3 | triglycéride d'acides caprylique et caprique, |
| - Comparaison 4 | di-isopropyl adipate, |
| - Comparaison 5 | isodéyl laurate, |
| - Comparaison 6 | isopropyl myristate, |
| - Comparaison 7 | oléyl oléate, |
| - Comparaison 8 | alcool oléique, |
| - Comparaison 9 | squalène. |

Résultats

Les valeurs d'IDT ainsi que la viscosité dynamique (m Pa. s) et la thixotropie (Pa. s) des lotions sont indiquées dans le tableau 1 ci-après

Tableau 1

| Caractéristiques des lotions | | | |
|---|---|---|---|
| Composé | IDT | Viscosité | Thixotropie |
| Exemple 1 | 4,5/18 | 2226 | 1400 |
| Exemple 2 | 4,5/14 | 1711 | 2236 |
| Comparaison 1 | 3,5/16 | ---- | ---- |
| Comparaison 2 | 4,5/18,5 | 1734 | 1852 |
| Comparaison 3 | 3,5/15 | 1958 | 1773 |
| Comparaison 4 | 3,5/18 | 2399 | 2743 |
| Comparaison 5 | 4 /13 | 1801 | 2128 |
| Comparaison 6 | 4,5/17,5 | 1829 | 1877 |
| Comparaison 7 | 4 /15 | 1801 | 1368 |
| Comparaison 8 | 4 /11,5 | 2668 | 2601 |
| Comparaison 9 | 4 /17,5 | 1409 | 1573 |

Les résultats précédents montrent les qualités organoleptiques des composés utilisables selon l'invention :

Par rapport aux représentants des différentes classes d'émollients, les composés des exemples 1 et 2 confèrent un toucher initial riche (impression de satiné) ainsi qu'une sensation extrêmement agréable sur la peau par leur excellente qualité d'étalement.

Le composé de l'exemple 1 en particulier atteint le meilleur score, tandis que la valeur de l'IDT du composé de l'exemple 2 se situe dans une très bonne moyenne, et en tout cas très nettement supérieure à celle des composés du type des alcools gras et fractions de triglycérides utilisés également comme agents de viscosité (comparaisons 3 et 8).

Pour ce qui est des propriétés rhéologiques, le composé de l'exemple 1 présente des avantages par rapport à la plupart des émollients testés. En effet, on constate que l'incorporation d'esters ou de triglycérides modifie habituellement la viscosité des formules et cette modification doit être compensée par l'addition de produits tels que les alcools gras et les fractions de triglycérides qui ont une influence négative sur le toucher. De plus, sa thixotropie est très satisfaisante, ce qui dispense de l'adjonction de régulateurs.

Par ailleurs, le composé de l'exemple 2 (ou les autres esters de 2,3-butane diol avec les acides gras saturés myristique, palmitique et stéarique de hauts points de fusion) peut être utilisé avec succès comme agent de viscosité et émollient. On constate en effet que sa viscosité est satisfaisante malgré l'absence d'agents de viscosité tels que l'alcool cétéarylique et le stéarate de glycérol.

Exemples 3-9

3. Lait démaquillant (émulsion huile-dans-l'eau)

%

**PHASE A (lipidique)** 21,55

%

| | |
|---|---|
| Composé de l'exemple 1 | 7 |
| 2-éthyl-hexyl-2-éthyl hexanoate | 3 |
| Glycéryl tri-C10-18 acides | 4 |
| Huile de paraffine | 3 |
| Stearate de glycérol | 3 |
| Acide stearique | 1,5 |
| Tocophérol, butylhydroxy-anisole (BHA) et triéthyl citrate | 0,05 |

**PHASE C (aqueuse)** 48,07

| | |
|---|---|
| Eau | 47,88 |
| Tétrahydroxypropyl éthylène-diamine | 0,14 |
| EDTA disodique | 0,05 |

**ADDITIFS** 30,38

| | |
|---|---|
| Hydroxyéthylcellulose (solution aqueuse à 2%) | 30 |
| Méthylchlorothiazolinone et méthylisothiazolinone | 0,08 |
| Parfum | 0,3 |

100

4. Crème hydratante (émulsion huile-dans-l'eau)

|  |  | % |
| --- | --- | --- |
| **PHASE A (lipidique)** |  | 26,05 |
| PEG-8 -C12-18 alkyl ester | 10 |  |
| Composé de l'exemple 1 | 7 |  |
| Isodécyl laurate | 5 |  |
|  |  | % |
| Cétéaryl alcool | 4 |  |
| Tocophérol, BHA et |  |  |
| triéthyl citrate | 0,05 |  |
| **PHASE C (aqueuse)** |  | 73,67 |
| Eau | 62,67 |  |
| PEG-5 -C12-18 alcools | 2 |  |
| Propylène glycol | 5 |  |
| Panthénol | 2 |  |
| Sodium PCA | 2 |  |
| **ADDITIFS** |  | 0,28 |
| Parfum | 0,2 |  |
| Méthylchlorothiazolinone et |  |  |
| méthylisothiazolinone | 0,08 |  |
|  |  | 100 |

8

5. Crème de soins (émulsion eau-dans-l'huile)

|  | % |
| --- | --- |
| **PHASE A (lipidique)** | 39 |
| | |
| PEG-1 glycéryl oléostéarate et | |
| cire de paraffine | 12 |
| Huile de paraffine | 13 |
| Composé de l'exemple 1 | 8 |
| Triglycérides d'acides caprylique | |
| et caprique | 5 |
| 2-phénoxyéthanol, méthyl parabène, | |
| éthylparabène, propylparabène et | |
| butylparabène | 1 |

|  |  | % |
| --- | --- | --- |
| **PHASE C (aqueuse)** | | 60,8 |
| | | |
| Eau | 58,1 | |
| Sulfate de magnésium | | |
| heptahydrate | 0,7 | |
| Glycérine | 2 | |
| | | |
| **ADDITIF** | | 0,2 |
| | | |
| Parfum | 0,2 | |
| | | 100 |

6. Huile pour le visage et le corps (anhydre)

|  | % |
|---|---|
| Huile minérale | 56,85 |
| Composé de l'exemple 1 | 10 |
| Octyl octanoate | 10 |
| Triglycérides d'acides gras en C10 - C18 | 10 |
| Cyclométhicone | 5 |
| Isodécyl laurate | 5 |
| Octyl méthoxycinnamate | 3 |
| Parfum | 0,1 |
| Tocophérol, triéthyl citrate et BHA | 0,05 |
|  | 100 |

7. Baume anydre

|  | % |
|---|---|
| Paraffine | 4 |
| Ozokérite | 5 |
| 2-éthyl-hexyl-2-éthyl hexanoate | 45,6 |
| Composé de l'exemple 1 | 40 |
| Isodécyl laurate | 5 |
| Tocophérol, triéthyl citrate et BHA | 0,1 |
| Parfum | 0,3 |
|  | 100 |

On mélange les composants à environ 75°C.

8. Shampooing traitant (émulsion huile-dans-l'eau)

$$\%$$

**PHASE A (lipidique)**

| | |
|---|---|
| Cocoamphoglycinate | 10 |
| Ammonium laureth sulfate | 7 |
| Ammonium lauryl sulfate | 3 |
| Cocamidopropyl bétaïne | 2 |
| Composé de l'exemple 1 | 0,5 |
| Panthénol | 0,5 |

**PHASE B (aqueuse)**

| | |
|---|---|
| Eau | 68,9 |
| Acrylates/stéareth-20 méthacrylate copolymère | 4 |
| Hydroxypropyl méthylcellulose | 2 |
| Polysorbate | 1 |
| Quaternium 23 (polymères quaternisés) | 0,5 |
| Acide citrique | 5,65 |
| Méthyldibromoglutaronitrile et 2-phénoxyéthanol | 0,20 |
| | 100 |

On mélange les composants de la phase A à température ambiante, puis on ajoute les composants de la phase B dans la phase A en brassant.

9. Rouge à lèvres (anhydre)

|  | % |
|---|---|
| Huile de ricin | 27,45 |
| Composé de l'exemple 2 | 30,5 |
| Cire d'abeilles | 10,5 |
| Cire de candelilla | 7,5 |
| Ozokérite | 5,5 |
| Lanolate d'isopropyle | 5 |
| Colorants | 13,55 |
|  | 100 |

Tous les produits cosmétiques des exemples 3-9 ont été testés et présentent une bonne stabilité à 20°C, 37°C et 47°C pendant 3 mois.

Ils ont de bonnes propriétés organoleptiques soit :

- les émulsions comme les produits anhydres sont homogènes, fins, lisses et brillants.

**Revendications**

1. Composition cosmétique ou dermatologique caractérisée par le fait qu'elle contient un diester de 2,3-butanediol et d'acide gras en C16 - C22 à titre d'agent de viscosité et d'émollient.

2. Composition selon la revendication 1, caractérisée par le fait que le diester est présent à une concentration de 0,5 à 80% en poids de la composition.

3. Composition selon la revendication 1, caractérisée par le fait qu'elle se présente sous forme d'une émulsion eau-dans-l'huile ou huile-dans-l'eau, qu'elle contient 1 à 20% en poids d'un agent émulsifiant et que la phase grasse contient 0,5 à 20% en poids de diester par rapport au poids total de l'émulsion.

4. Composition selon la revendication 1, caractérisée par le fait qu'elle se présente sous forme anhydre et que la phase grasse contient 10 à 80% en poids de diester par rapport au poids total de la composition.

5. Composition selon la revendicaiton 1, caractérisée par le fait qu'elle contient 0,02 à 0,2% en poids d'antioxydant.

6. Composition selon l'une des revendications 1 à 5, caractérisée par le fait qu'elle contient un diester de 2,3-butanediol et essentiellement des acides oléique, palmitique et stéarique.

7. Composition selon l'une des revendications 1 à 5, caractérisée par le fait qu'elle contient un diester de 2,3-butanediol et essentiellement des acides oléique, palmitique, stéarique et laurique.

**Claims**

1. A cosmetic or dermatological composition, characterized in that it contains a diester of 2,3-butanediol and a fatty acid, more particularly a $C_{16-22}$ fatty acid, as viscosity agent and emollient.

2. A composition as claimed in claim 1, characterized in that the diester is present in a concentration of 0.5 to 80% by weight, based on the composition as a whole.

3. A composition as claimed in claim 1, characterized in that it is in the form of a water-in-oil emulsion or oil-in-water emulsion, in that it contains 1 to 20% by weight of an emulsifier and in that the fatty phase contains 0.5 to 20% by weight diester, based on the total weight of the emulsion.

4. A composition as claimed in claim 1, characterized in that it is in anhydrous form and in that the fatty phase contains 10 to 80% by weight diester, based on the total weight of the composition.

12

5. A composition as claimed in claim 1, characterized in that it contains 0.02 to 0.2% by weight antioxidant.

6. A composition as claimed in any of claims 1 to 5, characterized in that it contains a diester of 2,3-butanediol and, essentially, oleic, palmitic and stearic acid.

7. A composition as claimed in any of claims 1 to 5, characterized in that it contains a diester of 2,3-butanediol and, essentially, oleic, palmitic, stearic and lauric acids.

**Patentansprüche**

1. Kosmetische oder dermatologische Zusammensetzung, dadurch gekennzeichnet, daß sie als Viskositäts- und Weichmachmittel einen Diester aus 2,3-Butandiol und C 16 - C 22 Fettsäure enthält.

2. Zusammensetzung nach Anspruch 1, dadurch gekennzeichnet, daß der Diester in einer Konzentration von 0,8 bis 80 Gew.-% der Zusammensetzung vorliegt.

3. Zusammensetzung nach Anspruch 1, dadurch gekennzeichnet, daß sie in Form einer Wasser-in-Öl- oder einer Öl-in-Wasser-Emulsion vorliegt, daß sie 1-20 Gew.-% eines Emulgators enthält und daß die Fettphase 0,5-20 Gew.-% Diester, bezogen auf das Gesamtgewicht der Emulsion, enthält.

4. Zusammensetzung nach Anspruch 1, dadurch gekennzeichnet, daß sie in einer wasserfreien Form vorliegt und daß die Fettphase 10 bis 80 Gew.-% Diester, bezogen auf das Gesamtgewicht der Zusammensetzung, enthält.

5. Zusammensetzung nach Anspruch 1, dadurch gekennzeichnet, daß sie 0,02 bis 0,2 Gew.-% Antioxidans enthält.

6. Zusammensetzung nach einem der Ansprüche 1 - 5, dadurch gekennzeichnet, daß sie einen Diester aus 2,3-Butandiol sowie im wesentlichen Ölsäure, Palmitinsäure und Stearinsäure enthält.

7. Zusammensetzung nach einem der Ansprüche 1 - 5, dadurch gekennzeichnet, daß sie einen Diester aus 2,3-Butandiol und im wesentlichen Ölsäure, Palmitinsäure, Stearinsäure und Laurinsäure enthält.